Europäisches Patentamt

European Patent Office

Office européen des brevets

(11) Publication number: **0 354 460 B1**

## EUROPEAN PATENT SPECIFICATION

(45) Date of publication of patent specification: **13.04.94**  (51) Int. Cl.5: **C07G 17/00**, //C07C319/04

(21) Application number: **89114260.6**

(22) Date of filing: **02.08.89**

(54) Process to produce organosulfur compounds.

(30) Priority: **03.08.88 US 227957**

(43) Date of publication of application:
**14.02.90 Bulletin  90/07**

(45) Publication of the grant of the patent:
**13.04.94 Bulletin  94/15**

(84) Designated Contracting States:
**AT BE CH DE ES FR GB GR IT LI LU NL SE**

(56) References cited:
EP-A- 0 122 654
US-A- 2 434 510
US-A- 4 119 549

(73) Proprietor: **PHILLIPS PETROLEUM COMPANY**
**5th and Keeler**
**Bartlesville Oklahoma 74004(US)**

(72) Inventor: **Roberts, John Scott**
**1505 Manor Drive**
**Bartlesville, OK 74006(US)**

(74) Representative: **Dost, Wolfgang,**
**Dr.rer.nat.,Dipl.-Chem. et al**
**Patent- und Rechtsanwälte,**
**Bardehle . Pagenberg . Dost . Altenburg .**
**Frohwitter . Geissler & Partner,**
**Postfach 86 06 20**
**D-81633 München (DE)**

Rank Xerox (UK) Business Services
(3.10/3.09/3.3.3)

## Description

Background of the Invention

This invention relates to a method for making organo-sulfur compounds.

It is known in the art to react olefins with hydrogen sulfide or mercaptans in the presence of various catalysts to form organo-sulfur compounds, such as sulfides or mercaptans. However, prior art processes do not have high yields of the desired product. Frequently, the reaction conditions used in prior art processes isomerize, or rearrange the olefin reactant. As a result of this rearrangement, the percent conversion of the olefin reactant to a sulfur-containing product can be high, but selectivity to the desired sulfur-containing product can be low. Olefins in the presence of acidic catalysts are especially subject to rearrangement. Some prior art processes require the use of ultraviolet or radioactive radiation to catalyze the reaction.

In US 4 119 549 a method is disclosed wherein sulfurized compositions are prepared by the reaction of unsaturated compounds and a mixture of sulfur and hydrogen sulfide under superatmospheric pressure and in the presence of a catalyst, for example an acidified clay or sodium hydroxide.

Summary of the Invention

It is an object of this invention to provide a novel process to prepare organo-sulfur compounds, wherein the use of a simple catalyst system, high product yield, and high product selectivity are subaspects.

In accordance with this invention, organo-sulfur compounds are prepared by reacting an aliphatic olefin having from 4 to 20 carbon atoms with a sulfur-containing compound selected from the group consisting of hydrogen sulfide and straight chain and branched aliphatic mercaptans having from 1 to 18 carbon atoms in the presence of a slightly acidic catalyst system comprising sodium hydroxide or one or more transition metals or transition metal oxides selected from the group consisting of vanadium, chromium, manganese, iron, nickel, copper, and zinc; and a modifier selected from the group consisting of molybdenum, molybdenum oxides, tungsten, tungsten oxides and mixtures thereof; all supported on an inorganic oxide support.

Reactants

Olefin, or alkene, compounds suitable for use in this invention to prepare organo-sulfur compounds are any straight chain or branched aliphatic olefin compounds having from 4 to 20 carbon atoms per molecule. Preferably, the olefin compound will have from 4 to 16 carbon atoms and most preferably from 8 to 16 carbon atoms per molecule. Olefins with a lower carbon content are usually easier to prepare or synthesize and, thus, are more readily available. Olefins with two or three carbon atoms, unlike olefins with about 4 or more carbon atoms, are not subject to isomerization, or rearrangement, during the course of the reaction.

The olefin reactant is also preferably an alpha-olefin because interferences in the reaction due to steric hindrance can be decreased. If the olefin reactant is branched, it is preferable to have the branching separated from the carbon double bond to further reduce problems that can be caused by steric hindrance. As used in this disclosure, the term "olefin" means an organic compound containing one carbon-carbon double bond.

Suitable olefin compounds include, but are not limited to, 1-butene, 2-butene, 1-pentene, 2-pentene, 2-methyl-1-butene, 2-methyl-2-butene, 3-methyl-1-butene, 2-methyl-1-pentene, 3-methyl-1-pentene, 4-methyl-1-pentene, 1-hexene, 2-hexene, 3-hexene, 2,3-dimethyl-1-butene, 2,3-dimethyl-2-butene, 2-ethyl-1-butene, 2-methyl-2-pentene, 3-methyl-2-pentene, 4-methyl-2-pentene, 1-heptene, 2-heptene, 3-heptene, 2-methyl-1-hexene, 3-methyl-1-hexene, 4-methyl-1-hexene, 5-methyl-1-hexene, 2-methyl-2-hexene, 3-methyl-2-hexene, 4-methyl-2-hexene, 5-methyl-2-hexene, 2-methyl-3-hexene, 3-methyl-3-hexene, 2,3-dimethyl-1-pentene, 2,4-dimethyl-1-pentene, 3,3-dimethyl-1-pentene, 3,4-dimethyl-1-pentene, 4,4-dimethyl-1-pentene, 2,3-dimethyl-2-pentene, 2,4-dimethyl-2-pentene, 3,4-dimethyl-2-pentene, 4,4-dimethyl-2-pentene, 2-ethyl-1-pentene, 3-ethyl-1-pentene, 3-ethyl-2-pentene, 2,3,3-trimethyl-1-butene, 2-ethyl-3-methyl-1-butene, 2-ethyl-3-methyl-2-butene, 1-octene, etc., 1-decene, 1-dodecene, 1-tetradecene, 1-hexadecene. Preferably 1-butene, 1-pentene, 1-hexene, 1-octene, 1-decene, 1-dodecene, 4-methyl-1-pentene, 2-methyl-1-butene are used for the reasons stated above.

Sulfur-containing compounds suitable for use in this invention are selected from the group consisting of hydrogen sulfide and straight chain or branched aliphatic mercaptans having from 1 to 18 carbon atoms. Preferably, if a mercaptan is used, the mercaptan has less than or equal to about 12 carbon atoms, and most preferably less than or equal to about 6 carbon atoms. Mercaptans having a lower molecular weight are easier to handle and are more easily separated from the desired reaction product.

Suitable sulfur-containing compounds include, but are not limited to methyl mercaptan, ethyl mercaptan, n-propyl mercaptan, isopropyl mercaptan, n-butyl mercaptan, 2-butanethiol, 2-methyl-1-propanethiol, 2-methyl-2-propanethiol, 1-pentanethiol, 2-methyl-1-pentanethiol, 3-methyl-1-pentanethiol, 4-methyl-1-pentanethiol, 2-pentanethiol, 3-pentanethiol, 2-methyl-2-pentanethiol, 3-methyl-2-pentanethiol, 4-methyl-2-pentanethiol, 2-methyl-3-pentanethiol, 3-methyl-3-pentanethiol, 2,3-dimethyl-1-butanethiol, 2,2-dimethyl-1-butanethiol, 3,3-dimethyl-1-butanethiol, 2,3-dimethyl-2-butenethiol, 1-octenethiol, t-nonyl mercaptan, 2,4,4-trimethyl-1-pentanethiol, 1-decanethiol, 1-dodecenethiol, 2-dodecenethiol, 5-dodecanethiol. Preferably, methyl mercaptans, ethyl mercaptan, n-propyl mercaptan, isopropyl mercaptan, n-butyl mercaptan, t-butyl mercaptans are used for the reasons stated above. The most preferred sulfur-containing compound useful in this invention is hydrogen sulfide because it is easily removed from the desired reaction product and can be used to aid heat removal from the reaction.

The reactants can be present in the reaction vessel in any quantity. For best reactant use and reaction rate efficiency, the reactants are combined in a molar ratio of sulfur-containing compound to olefin in the range of 1:1 to 10:1, preferably in the range of 2:1 to 6:1. Most preferably, the reactants are combined in a molar ratio of sulfur-containing compound to olefin in the range of 2:1 to 4:1 in order to optimize selectivity to the desired product. The inventive process proceeds very well at about a 3:1 molar ratio of the reactants.

The reactants can be added to the reaction vessel, or reactor, in any order. If desired, the reactants can be in a diluent; however, use of a diluent can decrease the reaction efficiency. If a diluent is desired, inert hydrocarbons are preferred. In the context of this invention, inert hydrocarbons are defined as anything that is not reducible by and will not react with the reactants or reaction products. Thus, compounds that are not reducible by and do not react with sulfur-containing compounds and/or olefins are acceptable diluents. Inert hydrocarbon diluents include, such as, for example, hexane, cyclohexane, and mixtures thereof. Examples of compounds which have a detrimental effect on the reaction and are, therefore, unacceptable diluents include, but are not limited to, water, alcohols, ketones, aldehydes, and chlorinated hydrocarbons.

Catalyst

The catalyst system useful in this invention is any slightly acidic inorganic oxide supported catalyst. Such catalysts are commercially available. Other catalyst systems, such as acidic inorganic oxide supported catalyst systems, can also be used to catalyze the reaction between an olefin and a sulfur-containing compound. However, catalyst systems other than slightly acidic inorganic oxide supported catalysts are not as highly selective to the desired product. Although not wishing to be bound by theory, it appears that acidic inorganic oxide supported catalyst systems appear to have a tendency to isomerize, or rearrange, the olefin reactant. Thus, the reaction products are not those specifically desired and the yield of the desired reaction product is lower than anticipated.

The term "inorganic oxide support" is intended to include non-acidic or slightly acidic inorganic oxide materials which have low double bond isomerization activity under the reaction conditions employed. Suitable materials include, but are not limited to, alumina, silica, silica-alumina, phosphated-alumina, magnesia, titania, magnesia-titania, thoria, zirconia, and mixtures thereof. Alumina and phosphated-alumina are preferred because of their ready availability, ease of handling, and resultant good catalyst activity.

The catalytic material deposited on the inorganic oxide support can be sodium hydroxide or one or more transition metals or transition metal oxides selected from the group consisting of vanadium, chromium, manganese, iron, cobalt, nickel, copper, and zinc; and a modifier selected from the group consisting of molybdenum, tungsten, molybdenum oxides, tungsten oxides, and mixtures thereof. The catalytic material, in combination with the inorganic oxide support, provides a catalyst system that is only slightly acidic.

Preferably, a cobalt/molybdate on phosphated-alumina is the catalyst system used for best selectivity to the desired reaction product. The amounts of cobalt and molybdate on the phosphated-alumina support are those sufficient to provide catalytic activity for the sulfidization reaction, yet still maintain a slightly acidic catalyst system. This preferred catalyst, based on the dry weight of the total catalyst system, preferably comprises cobalt, in the form of cobalt oxide (CoO), in the range of from 3 to 7 weight percent; molybdenum, in the form of molybdenum oxide ($MoO_3$), in the range of from 13 to 19 weight percent; supported on a phosphated-alumina support, wherein alumina is present in the range of from 75 to 80 weight percent, and phosphorus oxide is present in the range of from 1 to 4 weight percent.

3

Reaction Conditions

The sulfidization reaction can be carried out using either batch or continuous operation, although the invention is particularly well suited for continuous operation. Suitable equipment, such as reaction vessels, tubes, valves, and the like are well known in the art and can be employed. No special materials of construction for the reaction vessel are required, so that steel, stainless steel, glass-lined reactors, or the like can be employed. Preferably, to ensure complete contact of the reactant and catalyst, the reaction vessel is stirred or agitated. If a batch reactor is used, the vessel can be stirred or agitated by any suitable method. If a continuous process is employed, the flow of the reactant stream(s) over the catalyst can provide sufficient mixing.

The reaction temperature can vary, depending on the sulfur-containing compound and olefin employed. The reaction can proceed at any temperature. However, too low of a temperature can result in a low conversion rate of olefin to the desired reaction product. Too high of a temperature can result in high olefin conversion, but can also isomerize, or rearrange, the olefin to form undesired reaction products. Since the reaction is exothermic, the furnace, or inlet, or initial reaction temperature can vary from the outlet, or final, reaction temperature. Generally, the reaction vessel and reactants will be heated to an inlet, or initial, temperature in the range of 80 to 200°C, preferably in the range of 110 to 180°C. Most preferably, the initial reaction temperature is in the range of 140 to 160°C.

The pressure of the reaction vessel can vary, depending on the reaction conditions, as well as the reactants. The reaction can proceed at any pressure. The reaction vessel, if desired, can be pressurized up to about 6.9 MPa gauge (1,000 psig). Preferably, the reaction vessel pressure is in the range of 1.9 to 5.5 MPa gauge (280 to 800 psig), and most preferably in the range of 2.8 to 4.8 MPa gauge(400 to 700 psig). Low reaction zone pressures can result in slow reaction rates. High reaction vessel pressures can result in a more costly reaction vessel design.

Since the sulfidization reaction is exothermic, if desired, the reactor contents can be cooled during the course of the reaction by any method known in the art. For example, heat can be affirmatively removed by the use of cooling coils in and/or around the reaction vessel. Alternatively, if desired, the reactants can be used to effectuate the cooling. For example, if the sulfur-containing reactant is hydrogen sulfide, excess hydrogen sulfide gas can be used to dissipate some of the heat of the reaction.

The reaction preferably occurs in the absence of oxygen. For convenience, hydrogen sulfide or mercaptan is usually the ambient in the reaction vessel.

The contact time required for the sulfidization reaction depends upon several factors such as for example the activity of the catalyst, temperature, pressure, structure of the reactants employed and level of conversion desired. The length of time during which the olefin and sulfur-containing compounds are contacted with catalyst can vary conveniently between 0.1 s and 24 h although shorter and longer contact times can be employed. Preferably, times of one minute to 5 h are employed. Where reaction is carried out in continuous fashion, it is convenient to express the reactant/catalyst contact time in terms of weight hourly space velocity (WHSV), i.e., the ratio of the weight of reactant which comes in contact with a given weight of catalyst per unit time. Thus, a WHSV of 0.1 to 5 will be employed. A WHSV of 0.5 to 3 is preferred, with 1 to 2 WHSV most preferred for optimum catalyst productivity.

Under ideal conditions, high selectivity to the desired sulfur-containing product can be obtained. A relatively high product yield, with high product purity, can also be obtained. As used in this disclosure, selectivity, or product selectivity, is defined as the percent, by weight, of the desired reaction product in the total reaction product. Product yield is the percent, by weight, of actual yield of the desired reaction product based on the theoretical yield of the desired reaction product. Product selectivity can be over 70% and product yield can be over 60%.

Examples

Comparison I

The illustrative reaction was carried out with a stainless steel tubular reactor with 40.64 cm h x 2.54 cm O.D.(16"h x 1"O.D.) loaded with 83.6 grams (g) of catalyst Filtrol® 24 (available from Harshaw/Filtrol Partnership), which is acid-washed montmorillonite (an acid-washed silica-alumina clay) and is an acidic catalyst. The catalyst was loaded into the reactor on a small layer of glass wool and capped with a layer of glass wool.

A feed stream, containing 1-dodecene and hydrogen sulfide (the mole ratio of $H_2S$ to 1-dodecene was 3:1) was pumped from the top of the reactor to the bottom of the reactor, using a Lapp LS-20 pump, at a

4

rate of 3.07 grams/min, and a weight hour space velocity (WHSV) of 2.2 hr$^{-1}$, into the reactor. The reactor was controlled at 3.8 MPa gauge (560 psig) using a Moore flow controller. A three-zoned electrical furnace, regulated by Eurotherm temperature controller was set to 160°C. Three thermocouples were fixed at 33.02, 20.32 and 7.62 cm (13", 8" and 3") of a thermowell to monitor the temperatures of the reactor. When the feed reservoir containing 588.2g 1-dodecene and 356.8g hydrogen sulfide was emptied, the product was collected following the release of reactor pressure and unreacted $H_2S$ was vented to flare.

The product was distilled using an 45.7 x 4.4 cm (18" x 1.75") glass column that was packed with high efficiency stainless steel packing. The distillation temperature was 130-140°C and pressure was 68 Pa (5 mm Hg). The product was analyzed using a gas chromatograph equipped with an FID detector and a 12 m x 0.2 mm capillary column containing cross-linked methyl silicone. The oven temperature was programmed to ramp from 50° to 250°C at a ramping rate of 15°C/min. The injector temperature was 300°C.

Analysis showed the final product mixture, based on weight percent, contained 8.26% of unreacted 1-dodecene, representing a 90.25% conversion; 5.59% other dodecenes; 40.8% of 1-dodecyl mercaptan, representing a selectivity of 44.5%, 4.63% other dodecyl mercaptans and 32.8% sulfides. These data are raw, non-normalized analytical results.

Comparisons II-VI

The experiments were identical to that described in Comparison I except that the weight of reactants, furnace temperature and weight hour space velocity (WHSV) were varied. The raw, non-normalized analytical results are shown in Table I.

## TABLE I

### sec-Dodecyl Mercaptan Synthesis

Hydrogen Sulfide to:
1-Dodecene Mole Ratio: 3:1
Pressure: 3.8 Mpa gauge (560 psig)

| Run | 1-C12= (g)[a] | Furnace (°C) | WHSV (hr$^{-1}$) | Conversion (%)[b] | Selectivity (%) 2-C12SH[c] | C12SH[d] | Sulfide (%)[e] | Distilled Yield (%) | 2-C12SH Purity (%) |
|---|---|---|---|---|---|---|---|---|---|
| 2 | 667.8 | 141 | 2.53 | 25.1 | 19.8 | 49.0 | N.D.[f] | N.D. | N.D. |
| 3 | 322.9 | 171 | 1.52 | 87.2 | 13.8 | 61.0 | 24.8 | 39.5 | 31.6 |
| 4 | 364.1 | 160 | 1.40 | 93.6 | 14.4 | 63.0 | 30.3 | 33.4 | 32.6 |
| 5 | 386.5 | 160 | 1.41 | 95.1 | 28.3 | 61.0 | 15.3 | 36.2 | 30.7 |
| 6 | 292.5 | 160 | 0.68 | 95.9 | 31.4 | 68.0 | 16.3 | 52.7 | 31.2 |

a 1-C12=, 1-dodecene
b All compounds that were not 1-dodecene were considered as converted
c 2-C12SH, 2-dodecyl mercaptan
d C12SH, total dodecyl mercaptans
e Total sulfides
f N.D., not determined

The results shown in Table I indicate that 2-dodecyl mercaptan and other mercaptans can be readily prepared by the reaction of 1-dodecene and $H_2S$ over Filtrol® 24 with up to about 96% conversion of 1-dodecene. However, the selectivity to 2-dodecyl mercaptan is very low, less than 32%. Additionally, the yield and purity of desired 2-dodecyl mercaptan arc also low, less than 53% and 33% respectively. Thus, Filtrol® 24, an acidic catalyst, produces a large amount of isomerized olefins. To selectively produce such an isomer, a better system is needed.

6

Examples I-VI

A stainless steel tubular reactor, identical to that described in Comparison I, was loaded with 67g of HDS-20® catalyst (available from American Cyanamid Co. as, Trilobe® HDS-20®, which is a mixture of cobalt and molybdenum oxides on phosphated-alumina) that had been heated to 300°C in flowing nitrogen.

The reaction conditions were the same as those described in Comparisons II-VI except that the furnace temperature was maintained at 151°C, a 91.4 x 4.4 cm (36" x 1.75") glass column was used in distillation and a 50m x 0.2mm Hewlett-Packard Ultra-1® capillary column was used in gas chromatography. The raw, non-normalized analytical results are shown in Table II.

## TABLE II

### sec-Dodecyl Mercaptan Synthesis

Hydrogen Sulfide to:
1-Dodecene Mole Ratio:  3:1
Pressure: 3.8 Mpa gauge (560 psig)

| Run | 1-C12$^=$ (g)[a] | Furnace (°C) | WHSV (hr$^{-1}$) | Conversion (%)[b] | Selectivity (%) | | | Distilled Yield (%) | 2-C12SH Purity (%) |
|---|---|---|---|---|---|---|---|---|---|
| | | | | | 2-C12SH[c] | C12SH[d] | Sulfide (%)[e] | | |
| 1 | 254.6 | 151 | 0.85 | 72.7 | 33.6 | 75.9 | 16.7 | 62.4 | 88.9 |
| 2 | 212.2 | 151 | 1.00 | 77.5 | 45.3 | 77.4 | 13.8 | 57.6 | 90.3 |
| 3 | 214.7 | 151 | 1.02 | 70.6 | 73.9 | 81.5 | 36.7 | 56.3 | 89.3 |
| 4 | 254.6 | 151 | 1.06 | 88.7 | 68.1 | 76.4 | 13.2 | 53.8 | 91.1 |
| 5 | 388.4 | 151 | 1.30 | 76.8 | 67.6 | 75.7 | 14.8 | 60.0 | 88.0 |
| 6 | 492.0 | 151 | 1.69 | 87.0 | 69.2 | 77.5 | 15.7 | 60.9 | 91.1 |

[a] 1-C12$^=$, 1-dodecene

[b] All compounds that were not 1-dodecene were considered as converted

[c] 2-C12SH, 2-dodecyl mercaptan

[d] C12SH, total dodecyl mercaptans

[e] Total sulfides

Results presented in Table II suggest that a selectivity as high as 69.2% to 2-dodecyl mercaptan can be obtained using this inventive process. The selectivity is more than twice that shown in Table I. A much higher overall yield was also obtained using the HDS-20® catalyst system when compared to Filtrol® 24. Most strikingly, the product prepared from reactions in which HDS-20® was used had a purity of about 89 to about 91%, almost three times that shown in Table I.

8

**Claims**

1. A process for producing organo-sulfur compounds,
   **characterized by** reacting
   (a) an aliphatic olefin having from 4 to 20 carbon atoms per molecule with
   (b) a sulfur-containing compound selected from hydrogen sulfide and straight chain and branched aliphatic mercaptans having from 1 to 18 carbon atoms
   (c) in the presence of a slightly acidic catalyst system comprising sodium hydroxide or one or more transition metals or oxides of transition metals selected from vanadium, chromium, manganese, iron, nickel, coppper, and zinc; and a modifier selected from molybdenum, molybdenum oxides, tungsten, tungsten oxides and mixtures thereof; all supported on an inorganic oxide support.

2. The process of claim 1, wherein said olefin has from 4 to 16 carbon atoms.

3. The process of claim 1 or 2, wherein said olefin is an alpha-olefin.

4. The process of claim 2 or 3, wherein said olefin is 1-dodecene.

5. The process of any of the preceding claims, wherein said sulfur-containing compound has from 1 to 12 carbon atoms.

6. The process of any of claims 1 to 4, wherein said sulfur-containing compound is hydrogen sulfide.

7. The process of any of the preceding claims, wherein said inorganic oxide is selected from alumina, phosphated-alumina, silica-alumina, silica, magnesia, titania, magnesia-titania, thoria, zirconia, and mixtures of two or more thereof.

8. The process of any of the preceding claims, wherin said slightly acidic catalyst system comprises cobalt and molybdate on phosphated alumina.

9. The process of any of the preceding claims, being carried out continuously at a weight hourly space velocity in the range of 0.1 to 5.

10. The process of any of the preceding claims, wherein said reacting is carried out at a temperature of 80 to 300°C.

11. The process of any of the preceding claims, wherein said reacting is carried out under a pressure of about 6.9 MPa or below.

12. The process of any of the preceding claims, wherein the mole ratio of said sulfur-containing compound to said olefin is in the range of 1 : 1 to 10 : 1.

13. The process of claim 1 for producing 2-dodecane thiol comprising reacting
    (a) 1-dodecene with
    (b) hydrogen sulfide
    (c) in the presence of a slightly acidic catalyst system comprising cobalt and molybdate on phosphated alumina.

14. The process of claim 13, wherein said reacting is carried out at a temperature initially in the range of 140 to 160°C.

15. The process of claim 13 or 14, wherein said reacting is carried out at a pressure is in the range of 2.76 to 4.83 MPa.

16. The process of any of claims 13 to 15, wherein the mole ratio of 1-dodecene to hydrogen sulfide is in the range of 2 : 1 to 4 : 1.

# EP 0 354 460 B1

**Patentansprüche**

1.  Verfahren zur Herstellung von Organoschwefelverbindungen, gekennzeichnet durch Umsetzung
    (a) eines aliphatischen Olefins, das 4 bis 20 Kohlenstoffatome pro Molekül aufweist, mit
    (b) einer schwefelhaltigen Verbindung, die unter Schwefelwasserstoff und geradkettigen und verzweigten aliphatischen Mercaptanen mit 1 bis 18 Kohlenstoffatomen ausgewählt ist,
    (c) in Gegenwart eines leicht sauren Katalysatorsystems, das Natriumhydroxid oder ein oder mehrere Übergangsmetalle oder Oxide von Übergangsmetallen, die unter Vanadium, Chrom, Mangan, Eisen, Nickel, Kupfer und Zink ausgewählt sind; und ein modifizierendes Mittel, das unter Molybdän, Molybdänoxiden, Wolfram, Wolframoxiden und Gemischen davon ausgewählt ist, umfaßt; wobei sich alle auf einem anorganischen Oxidträger befinden.

2.  Verfahren nach Anspruch 1 wobei das Olefin 4 bis 16 Kohlenstoffatome aufweist.

3.  Verfahren nach einem der Ansprüche 1 oder 2, wobei es sich bei dem Olefin um ein $\alpha$-Olefin handelt.

4.  Verfahren nach einem der Ansprüche 2 oder 3, wobei es sich bei dem Olefin um 1-Dodecen handelt.

5.  Verfahren nach einem der vorstehenden Ansprüche, wobei die schwefelhaltige Verbindung 1 bis 12 Kohlenstoffatome aufweist.

6.  Verfahren nach einem der Ansprüche 1 bis 4, wobei es sich bei der schwefelhaltigen Verbindung um Schwefelwasserstoff handelt.

7.  Verfahren nach einem der vorstehenden Ansprüche, wobei das anorganische Oxid unter Aluminiumoxid, phosphatiertem Aluminiumoxid, Siliciumdioxid-Aluminiumoxid, Siliciumdioxid, Magnesiumoxid, Titandioxid, Magnesiumoxid-Titandioxid, Thoriumdioxid, Zirconiumdioxid und Gemischen von zwei oder mehr davon ausgewählt ist.

8.  Verfahren nach einem der vorstehenden Ansprüche, wobei das leicht saure Katalysatorsystem Cobalt und Molybdat auf phosphatiertem Aluminiumoxid umfaßt.

9.  Verfahren nach einem der vorstehenden Ansprüche, wobei das Verfahren kontinuierlich mit einer stündlichen Gewichts-Raumgeschwindigkeit im Bereich von 0,1 bis 5 durchgeführt wird.

10. Verfahren nach einem der vorstehenden Ansprüche, wobei die Umsetzung bei einer Temperatur von 80 bis 300 ° C durchgeführt wird.

11. Verfahren nach einem der vorstehenden Ansprüche, wobei die Reaktion bei einem Druck von etwa 6,9 MPa oder darunter durchgeführt wird.

12. Verfahren nach einem der vorstehenden Ansprüche, wobei das Molverhältnis der schwefelhaltigen Verbindung zu dem Olefin im Bereich von 1:1 bis 10:1 liegt.

13. Verfahren nach Anspruch 1 zur Herstellung von 2-Dodecanthiol, umfassend die Umsetzung von
    (a) 1-Dodecen mit
    (b) Schwefelwasserstoff
    (c) in Gegenwart eines leicht sauren Katalysatorsystems, das Cobalt und Molybdat auf phosphatiertem Aluminiumoxid umfaßt.

14. Verfahren nach Anspruch 13, wobei die Umsetzung bei einer Temperatur, die zu Anfang im Bereich von 140 bis 160 ° C liegt, durchgeführt wird.

15. Verfahren nach Anspruch 13 oder 14, wobei die Umsetzung bei einem Druck im Bereich von 2,76 bis 4,83 MPa durchgeführt wird.

16. Verfahren nach einem der Ansprüche 13 bis 15, wobei das molare Verhältnis von 1-Dodecen zu Schwefelwasserstoff im Bereich von 2:1 bis 4:1 liegt.

EP 0 354 460 B1

**Revendications**

1. Un procédé pour produire des composés de soufre organiques caractérisé en ce qu'il consiste à faire réagir:
   (a) une oléfine aliphatique ayant de 4 à 20 atomes de carbone par molécule avec
   (b) un composé contenant du soufre choisi parmi l'hydrogène sulfuré et les mercaptans aliphatiques à chaîne droite et ramifiée ayant de 1 à 18 atomes de carbone,
   (c) en présence d'un système de catalyseur légèrement acide comprenant de l'hydroxyde de sodium ou un ou plusieurs métaux de transition ou des oxydes de métaux de transition choisis parmi la vanadium, le chrome, le manganèse, le fer, le nickel, le cuivre et le zinc ; et un agent modificateur choisi parmi le molybdène, les oxydes de molybdène, le tungstène, les oxydes de tungstène et leurs mélanges, tous fixés sur un support d'oxyde minéral.

2. Le procédé selon la revendication 1, dans lequel ladite oléfine comprend de 4 à 16 atomes de carbone.

3. Le procédé selon la revendication 1 ou 2, dans lequel ladite oléfine est une alpha-oléfine.

4. Le procédé selon la revendication 2 ou 3, dans lequel ladite oléfine est le 1-dodécène.

5. Le procédé selon l'une quelconque des revendications précédentes dans lequel ledit composé contenant du soufre comprend de 1 à 12 atomes de carbone.

6. Le procédé selon l'une quelconque des revendications 1 à 4, dans lequel ledit composé contenant du soufre est de l'hydrogène sulfure.

7. Le procédé selon l'une quelconque des revendications précédentes dans lequel ledit oxyde minéral est choisi parmi l'alumine, l'alumine phosphatée, la silice-alumine, la silice, la magnésie, l'oxyde de titane, la magnésie-oxyde de titane, la thorie, l'oxyde de zirconium et les mélanges de deux ou plusieurs de ceux-ci.

8. Le procédé selon l'une quelconque des revendications précédentes dans lequel ledit système de catalyseur légèrement acide comprend du cobalt et du molybdate fixés sur de l'alumine phosphatée.

9. Le procédé selon l'une quelconque des revendications précédentes qui est mis en oeuvre de façon continue à une vitesse spatiale horaire pondérale comprise dans la gamme de 0,1 à 5.

10. Le procédé selon l'une quelconque des revendications précédentes dans lequel la réaction est mise en oeuvre a une température de 80 à 300°C.

11. Le procédé selon l'une quelconque des revendications précédentes dans lequel la réaction est mise en oeuvre sous une pression d'environ 6,9 MPa ou en dessous.

12. Le procédé selon l'une quelconque des revendications précédentes dans lequel le rapport molaire dudit composé contenant du soufre à ladite oléfine est compris dans la gamme de 1:1 à 10:1.

13. Le procédé selon la revendication 1, pour produire du 2-dodécane-thiol qui consiste à faire réagir:
    (a) du 1-dodécène avec
    (b) de l'hydrogène sulfuré
    (c) en présence d'un système de catalyseur légèrement acide comprenant du cobalt et du molybdate fixés sur de l'alumine phosphatée.

14. Le procédé selon la revendication 13, dans lequel ladite réaction est mise en oeuvre à une température comprise initialement dans la gamme de 140 à 160°C.

15. Le procédé selon la revendication 13 ou 14, dans lequel ladite réaction est mise en oeuvre à une pression comprise dans la gamme de 2,76 à 4,83 MPa.

11

16. Le procédé selon l'une quelconque des revendications 13 à 15 dans lequel le rapport molaire du 1-dodécène à l'hydrogène sulfuré est compris dans la gamme de 2:1 à 4:1.